# EUROPEAN PATENT APPLICATION

(11) **EP 2 450 391 A1**
(43) Date of publication of application: **09.05.2012**
(21) Application number: 11187759.3
(22) Date of filing: 03.11.2011
(51) Int. Cl.: C08G 63/78, C08J 11/10, C07D 319/00, B01J 19/00

(54) **System and method for producing polyhydroxycarboxylic acid**

(30) Priority: 04.11.2010 JP 2010247481
(71) Applicant: Hitachi Plant Technologies, Ltd., Tokyo 170-8466 (JP)
(72) Inventor: Kamikawa, Masayuki, Tokyo 100-8220 (JP); Matsuo, Toshiaki, Tokyo 100-8220 (JP); Okamoto, Naruyasu, Tokyo 170-8466 (JP); Oka, Kenichiro, Tokyo 100-8220 (JP); Kondo, Takeyuki, Tokyo 100-8220 (JP); Tomura, Akira, Tokyo 170-8466 (JP); Suzuki, Kazutaka, Tokyo 170-8466 (JP)
(74) Representative: Beetz & Partner

(57) **Abstract**

An object of the present invention is to provide a method and a system by which hydroxycarboxylic acid cyclic dimers are efficiently generated, allowing high yields of high-quality polyhydroxycarboxylic acid to be obtained. The method for synthesizing polyhydroxycarboxylic acid comprises a depolymerization step for depolymerizing hydroxycarboxylic acid oligomers to produce hydroxycarboxylic acid cyclic dimers, wherein, in the depolymerization step, a reaction solution is heated via heat transfer from a heat medium passage under reduced pressure while the reaction solution is being flowing through a horizontally provided reaction solution passage.

## Description

### BACKGROUND OF THE INVENTION

### Field of the Invention

The present invention relates to a system and a method for producing polyhydroxycarboxylic acid, and a system for producing hydroxycarboxylic acid cyclic dimers.

### Background Art

Polyhydroxycarboxylic acid is an aliphatic polyester produced by polymerizing hydroxycarboxylic acid. A typical example thereof is polylactide.

Examples of known methods for synthesizing polylactide include methods comprising a step of concentrating lactic acid as a raw material so as to reduce the water content (concentration step), a step of condensing lactic acid to generate oligomers (condensation step), a step of depolymerizing such oligomers to generate cyclic dimers (depolymerization step), and a step of subjecting cyclic dimers to ring-opening polymerization (ring-opening polymerization step). Polyhydroxycarboxylic acid other than polylactide can also be produced by a technique similar to the above method.

In the concentration step, water contained in the raw material is removed in order to facilitate the initiation of esterification between lactic acid molecules. In the condensation step, oligomers are generated during the removal of water resulting from esterification under heating and depressurization.

In the depolymerization step, in the presence of a depolymerization catalyst such as tin octylate as well as under heating and reduced pressure, oligomers are depolymerized and thus lactides, which are cyclic dimer esters of lactic acid, are generated. Lactides are generally in gaseous form under the environment of the depolymerization step. Hence, lactides are cooled, condensed, and then recovered. The thus obtained lactides are purified if necessary and then transferred to the ring-opening polymerization step.

A tank-type reaction container is often used in the depolymerization step. WO 93/15127 discloses a method for continuously producing lactides and lactide polymers, illustrating a tank-type container as a reaction container for generation of lactides.

### SUMMARY OF THE INVENTION

When a conventional tank-type reaction container is used in the depolymerization step, problems occur such that the viscosity of a reaction solution increases with time and the lactide yield decreases. Moreover, it is problematic that the optical purity of lactides tends to decrease for reasons such that pressure to be applied to the reaction solution increases as an increase in the liquid depth, for example. The lowered optical purity of lactides must be avoided as far as possible, since it results in lowered quality of polylactide as the final product. Therefore, an object of the present invention is to provide a method and a system by which high yields of high-quality polyhydroxycarboxylic acid can be obtained by efficiently generating hydroxycarboxylic acid cyclic dimers such as lactides.

The present inventors have discovered that hydroxycarboxylic acid cyclic dimers such as lactides can be efficiently generated while preventing optical purity from decreasing through a depolymerization step by which depolymerization is performed while causing a reaction solution to flow horizontally. The present invention is as summarized below.
(1) A method for synthesizing polyhydroxycarboxylic acid comprising a depolymerization step for depolymerizing hydroxycarboxylic acid oligomers to produce hydroxycarboxylic acid cyclic dimers, wherein,
   in the above depolymerization step, a reaction solution is heated by heat transfer from a heat medium passage under reduced pressure while the reaction solution is being flowing through a horizontally provided reaction solution passage.
(2) The method for synthesizing polyhydroxycarboxylic acid according to (1), wherein, in the above depolymerization step, the remaining solution that is discharged from an outlet of the reaction solution passage is recovered in a catch pot and at least a portion of the solution is refluxed to the reaction solution passage.
(3) A polyhydroxycarboxylic acid synthesis system comprising a depolymerization apparatus for depolymerizing hydroxycarboxylic acid oligomers to produce hydroxycarboxylic acid cyclic dimers, wherein
   the above depolymerization apparatus has a horizontally provided reaction solution passage, a heat medium passage that is in contact with the reaction solution passage, and a depressurization apparatus for depressurizing the reaction solution passage.
(4) The polyhydroxycarboxylic acid synthesis system according to (3), wherein, in the above depolymerization apparatus, a plurality of reaction solution passages are provided in parallel, and the heat medium passage is provided so as to enclose the individual reaction solution passages.
(5) The polyhydroxycarboxylic acid synthesis system according to (3), wherein, in the above depolymerization apparatus, a plurality of heat medium passages are provided within the reaction solution passage.
(6) The polyhydroxycarboxylic acid synthesis system according to any one of (3) to (5), wherein, in the above depolymerization apparatus, the reaction solution passage and the heat medium passages are provided in parallel.
(7) The polyhydroxycarboxylic acid synthesis system according to any one of (3) to (6), wherein, in the above depolymerization apparatus, a catch pot equipped with a liquid-level meter is provided at an outlet of the reaction solution passage.
(8) The polyhydroxycarboxylic acid synthesis method according to (1) or (2), wherein the system according to any one of (3) to (7) is used.
(9) A system for producing hydroxycarboxylic acid cyclic dimers, having a horizontally provided reaction solution passage, a heat medium passage that is in contact with the reaction solution passage, and a depressurization apparatus for depressurizing the reaction solution passage.
(10) The system for producing hydroxycarboxylic acid cyclic dimers according to (9), wherein a plurality of reaction solution passages are provided in parallel, and a heat medium passage is provided so as to enclose the individual reaction solution passages.
(11) The system for producing hydroxycarboxylic acid cyclic dimers according to (9), wherein a plurality of the heat medium passages are provided within the reaction solution passage.
(12) The system for producing hydroxycarboxylic acid cyclic dimer according to any one of (9) to (11), wherein the reaction solution passages and the heat medium passages are provided in parallel.
(13) The system for producing hydroxycarboxylic acid cyclic dimers according to any one of (9) to (12), wherein a catch pot equipped with a liquid-level meter is provided at an outlet of the reaction solution passage.

According to the system and the method of the present invention, no pressure is applied to a reaction solution since the reaction solution is depolymerized while the reaction solution is being flowing horizontally. Therefore, it becomes possible to efficiently generate hydroxycarboxylic acid cyclic dimers such as lactides and to obtain high yields of high-quality polyhydroxycarboxylic acid. Also, according to the method and the system of the present invention, reduction of the time for depolymerization reaction, a downsized depolymerization apparatus, and the like can be realized. The method and the system of the present invention are particularly appropriate for continuously performing a depolymerization step.

This specification incorporates the content of the specification of Japanese Patent Application No. 2010-247481, for which priority is claimed to the present application.

### BRIEF DESCRIPTION OF THE DRAWINGS

Fig. 1 shows an example of the system for producing polyhydroxycarboxylic acid according to the present invention.
Fig. 2 shows a depolymerization reactor, a catch pot, and peripheral apparatuses thereof.
Fig. 3 shows examples of the disposition of reaction solution passages and heat medium passages in depolymerization reactors.

### Explanation of Reference Numerals

1: Lactic acid feed apparatus, 2: Solution transfer pump, 3: Lactic acid concentration apparatus, 4: Solution transfer pump, 5: Concentrated lactic acid buffer tank, 6: Solution transfer pump, 7: Lactic acid condensation apparatus, 8: Solution transfer pump, 9: Oligomer buffer tank, 10: Solution transfer pump, 11: Oligomer feed pipe, 12: Reaction solution feed pipe, 13: Mixer, 14: Check valve, 15: Depolymerization reactor, 16: Catch pot, 17: Reflux pipe, 18: Drainage pipe, 19: Distilling column, 20: Distilling column lower level, 21: Solution transfer pump, 22: Lactide purification apparatus, 23: Solution transfer pump, 24: Ring-opening polymerization apparatus, 25: Refluxer, 26: Cooler, 27: Depressurization apparatus, 28: Refluxer, 29: Cooler, 30: Depressurization apparatus, 31: Condenser, 32: Cooler, 33: Depressurization apparatus, 34: Depolymerization residue buffer tank, 35: Decomposition apparatus for decomposing residue resulting from depolymerization, 36: Buffer tank, 37: Catalytic separation apparatus, 38: Catalytic removal apparatus, 39: Water separation apparatus, 40: Refluxer, 41: Cooler, 42: Water supply apparatus

### DESCRIPTION OF THE PREFERRED EMBODIMENTS

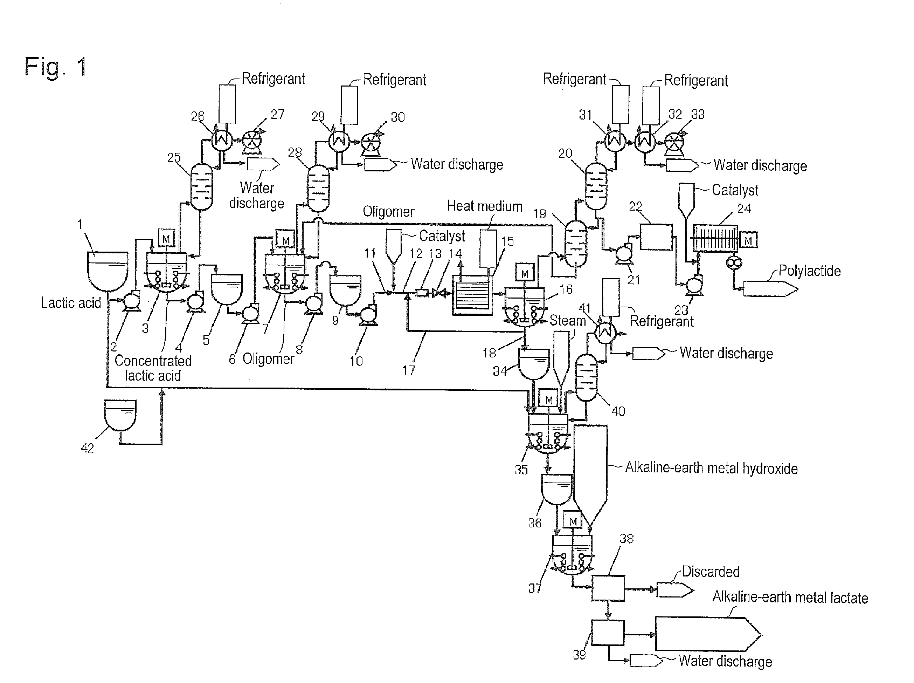

Fig. 1 shows an example of the system for producing polyhydroxycarboxylic acid of the present invention. For convenience, production of polylactide with the use of the system in Fig. 1 is described herein. However, the system can be used not only for polylactide production but also for production of other polyhydroxycarboxylic acids. Examples of other hydroxycarboxylic acids include glycolic acid, 3-hydroxybutyric acid, 4-hydroxybutyric acid, 4-hydroxyvaleric acid, 5-hydroxyvaleric acid, and 6-hydroxycaproic acid. When a polyhydroxycarboxylic acid other than polylactide is produced, the terms "lactide," "lactic acid oligomer," and "lactic acid" used in the descriptions below can be read as "hydroxycarboxylic acid cyclic dimer," "hydroxycarboxylic acid oligomer," and "hydroxycarboxylic acid," respectively. In addition, the term "depolymerization (reaction)" used herein refers to the generation of hydroxycarboxylic acid cyclic dimer (cyclic ester generated by a dehydration reaction for removing two water molecules from two hydroxycarboxylic acid molecules) from hydroxycarboxylic acid oligomers. Therefore, the term "depolymerization apparatus" may also refer to an apparatus for producing hydroxycarboxylic acid cyclic dimers.

In the lactic acid concentration apparatus 3, water contained in lactic acid is evaporated by heating (lactic acid concentration step). Heating is preferably carried out within a nitrogen gas flow at 120°C to 150°C. Water and lactic acid in gaseous form are generated from the lactic acid concentration apparatus 3. These gases enter a refluxer 25 and only lactic acid is refluxed to the lactic acid concentration apparatus 3. The thus obtained concentrated lactic acid is transferred to a lactic acid condensation apparatus 7 via a concentrated lactic acid buffer tank 5.

In the lactic acid condensation apparatus 7, a lactic acid condensation reaction is allowed to proceed for lactic acid oligomer generation (lactic acid condensation step). Water generated during the reaction is evaporated. The term "lactic acid oligomer" used in the specification refers to a lactic acid oligomer ranging from a lactic acid dimer to a lactic acid polymer having a weight average molecular weight of approximately 50,000 at maximum. The weight average molecular weight of lactic acid oligomers obtained as a result of the above lactic acid condensation reaction generally ranges from 150 to 10,000 and preferably ranges from 500 to 5,000. The lactic acid condensation reaction is preferably carried out at a reduced pressure of 10 torr or less at temperatures ranging from 120°C to 250°C. If necessary, catalysts such as organotin-based compounds (e.g., tin lactate, tin tartrate, tin dicaprylate, tin dilaurate, tin dipalmitate, tin distearate, tin dioleate, tin α-naphthoate, tin β-naphthoate, and tin octylate) or powdered tin may be used. Water, lactic acid, low-molecular-weight oligomer, and lactides are generated in gaseous form from the lactic acid condensation apparatus 7. These gases enter the refluxer 28 and then components other than water are refluxed to the lactic acid condensation apparatus 7. The thus obtained oligomer is transferred to a depolymerization reactor 15 via an oligomer feed pipe 11 and a reaction solution feed pipe 12. In addition, when a lactic acid oligomer is used as a raw material, the lactic acid concentration apparatus 3 and the lactic acid condensation apparatus 7 can be omitted.

In a depolymerization reactor 15, in the presence of a catalyst such as tin octylate, lactic acid oligomers are depolymerized to generate lactides that are cyclic dimers of lactic acid (depolymerization step). Crude lactides generated in gaseous form are transferred to a distilling column 19 via a catch pot 16. Residues resulting from depolymerization are stored in the catch pot 16. The depolymerization reactor 15 and the catch pot 16 are as described in detail later. In addition, the depolymerization apparatus in the present invention is meant to comprise at least the depolymerization reactor 15.

In a distilling column 19, vapor containing lactides is cooled and then impurities such as oligomers are liquefied and removed. Gaseous lactides from which impurities have been removed are transferred to a distilling column lower level 20. Since liquefied impurities contain high-level oligomers having molecular weights lowered as a result of depolymerization, the liquefied impurities are preferably refluxed to the lactic acid condensation apparatus 7 and reused as raw materials.

In the distilling column lower level 20, gaseous lactides are cooled and condensed, and then transferred to a lactide purification apparatus 22. Gases containing much water vapor separated from lactides are transferred to a condenser 31, so that lactides, lactic acid, low-molecular-weight oligomers, and the like are removed from the gases and then the resultant is refluxed to the distilling column lower level 20. Vapor that has not been condensed in the condenser 31 is liquefied in an impurity cooler 32 and then discarded. Gases that have not been condensed in the impurity cooler 32 are released to the outside of the system via a depressurization apparatus 33.

A desired example of a distilling column is a surface condenser in which vapor and a refrigerant indirectly come into contact with each other via metal tubes. This is because direct contact of lactides and oligomers with a water-containing refrigerant results in acid generation due to decomposition. Acid serves as a catalyst to inhibit the progress of a ring-opening polymerization reaction. In addition, acid may cause corrosion of materials such as a distilling column. There are exceptional cases in which a refrigerant that is inert in the presence of lactides and oligomers is used. In such a case, it is necessary to sufficiently dry a refrigerant to reduce the moisture therein.

The lactide purification apparatus 22 is used for separating target lactides (mainly L-lactide) from the other impurities for purification. Solvent extraction, melt crystallization, or the like can be used as a purification method. For example, such a melt crystallization method involves cooling melted crude lactides for partial crystallization, forming a solid crystalline phase with low amounts of impurities and a liquid phase with high amounts of impurities, separating the crystalline phase from the liquid phase, causing the crystalline phase to undergo sweating, and thus removing impurities. Then, lactides discharged from the lactide purification apparatus 22 are transferred to a ring-opening polymerization apparatus 24.

In the ring-opening polymerization apparatus 24, lactides are subjected to ring-opening polymerization under reduced pressure of about 10 torr or less at temperatures ranging from 120°C to 250°C (ring-opening polymerization step). A ring-opening polymerization catalyst and a polymerization initiator are preferably used for ring-opening polymerization of lactides.

As ring-opening polymerization catalysts, compounds comprising metals belonging to Groups IA, IIIA, IVA, IIB, and VA of the periodic table can be used.

Examples of catalysts comprising metals belonging to Group IA include hydroxides of alkali metals (e.g., sodium hydroxide, potassium hydroxide, and lithium hydroxide), salts of alkali metals and weak acids (e.g., sodium lactate, sodium acetate, sodium carbonate, sodium octylate, sodium stearate, potassium lactate, potassium acetate, potassium carbonate, and potassium octylate), alkoxides of alkali metals (e.g., sodium methoxide, potassium methoxide, sodium ethoxide, and potassium ethoxide).

Examples of catalysts comprising metals belonging to Group IIIA include aluminium ethoxide, aluminium isopropoxide, alumina, and aluminium chloride.

Examples of catalysts comprising metals belonging to Group IVA include organotin-based compounds (e.g., tin lactate, tin tartrate, tin dicaprylate, tin distearate, tin dioleate, tin α-naphthoate, tin (3-naphthoate, and tin octylate), powdered tin, oxidized tin, and halogenated tin.

Examples of catalysts comprising metals belonging to Group IIB include zinc powder, halogenated zinc, oxidized zinc, and organozinc-based compounds.

Examples of catalysts comprising metals belonging to Group IVB include titanium-based compounds such as tetrapropyl titanate and zirconium-based compounds such as zirconiumisopropoxide.

Among the above, it is preferable to use a tin-based compound such as tin octylate or an antimony-based compound such as antimony trioxide. In addition, the amount of such a catalyst to be used herein ranges from approximately 1 ppm to 2000 ppm, particularly preferably ranges from 5 ppm to 1500 ppm, and more particularly preferably ranges from 10 ppm to 1000 ppm with respect to lactide.

As polymerization initiators, for example, alcohols such as 1-dodecanol or compounds having other hydroxy groups can be used.

As the ring-opening polymerization apparatus 24, a vertical reactor, a horizontal reactor, or a tank-type reactor can be used. Two or more reactors may be aligned in series. Examples of an agitating blade that can be used include paddle blades, turbine blades, anchor blades, double-motion blades, and helical ribbon blades.

In the decomposition apparatus 35 for decomposing residue resulting from depolymerization, the residue resulting from depolymerization is hydrolyzed under pressurization at temperatures ranging from 120°C to 250°C through addition of steam. For improvement in the mixing properties of the residue resulting from depolymerization, it is desirable to maintain the melted state of the residue resulting from depolymerization. Hydrolysis is carried out under pressurization using a pressure-proof container (e.g., autoclave) as a reaction container, so that decomposition can take place while the melted state of the residue resulting from depolymerization is maintained. Alternatively, hydrolysis can also be carried out, following which the residue resulting from depolymerization is solidified and then pulverized for powderization. A residue containing lactic acid or low-molecular-weight oligomers resulting from hydrolysis is transferred to a catalytic separation apparatus 37 via a buffer tank 36.

In the catalytic separation apparatus 37, a catalyst contained in the residue is degraded and separated through addition of an alkaline-earth metal hydroxide such as calcium hydroxide or magnesium hydroxide. For example, when tin octylate is used as a catalyst, it is degraded in an inert gas atmosphere generally at temperatures ranging from 0°C to 250°C and preferably ranging from 5°C to 130°C through addition of calcium hydroxide. Thus, tin octylate is degraded to oxidized tin. The oxidized tin is insoluble to water or lactic acid and thus is precipitated. A residue containing the degraded catalyst is transferred to a catalytic removal apparatus 38.

In the catalytic removal apparatus 38, the degraded catalyst is removed from the residue using a filtration apparatus. Subsequently, the residue is transferred to a water separation apparatus 39, water is separated therefrom, and then the resultant is subjected to secondary use as a fertilizer such as lactate (e.g., calcium lactate) or discarded.

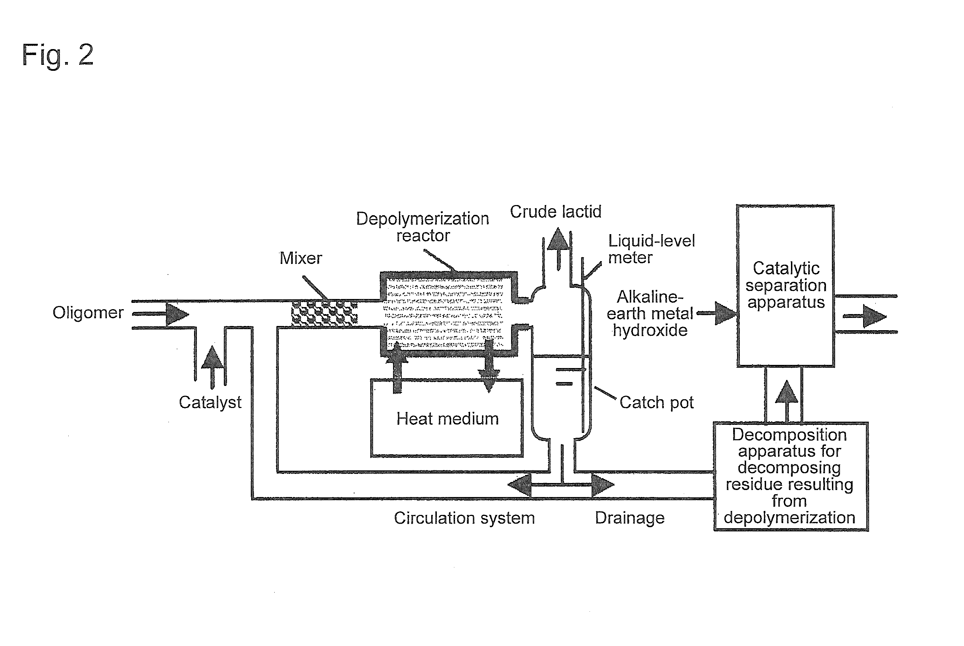

Fig. 2 shows the depolymerization apparatus of the present invention comprising a depolymerization reactor 15 and a catch pot 16, as well as the peripheral apparatuses. First, a catalyst for depolymerization is added to oligomers that are transferred from the lactic acid condensation apparatus 7. As catalysts for depolymerization reactions, compounds comprising metals belonging to Group IVA of the periodic table can be used, for example. Specific examples of catalysts for depolymerization reactions include organotin-based compounds (e.g., tin lactate, tin tartrate, tin dicaprylate, tin distearate, tin dioleate, tin α-naphthoate, tin β-naphthoate, tin octylate, and tin 2-ethylhexanoate). A catalyst is preferably used herein in an amount ranging from 0.01% by weight to 20% by weight, particularly ranging from 0.05% by weight to 15% by weight, and particularly preferably ranging from 0.1% by weight to 10% by weight with respect to that of an oligomer.

A reaction solution prepared by mixing an oligomer with a catalyst using a mixer is supplied to the depolymerization reactor 15. The depolymerization reactor 15 has a horizontally provided reaction solution passage and a heat medium passage that is in contact with the reaction solution passage. The reaction solution passage is connected to a depressurization apparatus 33 (not shown in Fig. 2) via the catch pot 16. The reaction solution is heated by heat transfer from the heat medium passage while the reaction solution is being flowing horizontally through the reaction solution passage, and then subjected to depolymerization. The reaction solution is heated in general at temperatures ranging from 120°C to 250°C and preferably ranging from 120°C to 200°C generally under reduced pressure of 100 torr or less and preferably 10 torr or less.

When excessive pressure is applied to a reaction solution, it is difficult to maintain a depressurization state, causing a problem such as inhibition of lactide generation. The reaction solution passage is horizontally provided, so that no pressure is applied to the reaction solution unlike a case in which a vertical interval is present in the reaction solution passage. Hence, such a horizontally provided reaction solution is preferable. In addition, the term "horizontal" in the specification is meant to include an incline of up to ±1° with respect to the horizontal surface.

The catch pot 16 is provided at an outlet of the reaction solution passage. A crude lactide discharge port is provided in the upper part of the catch pot 16. The crude lactide discharge port is connected to a distilling column 19. The residue generated by depolymerization is stored in the catch pot 16. A residue discharge port is provided in the lower part of the catch pot 16, and a portion of the discharged residue is circulated to the depolymerization reactor 15 via a reflux pipe. Circulation of such a residue to the depolymerization reactor 15 is preferable since it leads to an improved lactide yield. A residue portion that has remained uncirculated is discharged to the decomposition apparatus 35 for decomposing residue resulting from depolymerization via a drainage pipe.

A liquid-level meter is preferably provided in the catch pot 16, so that a liquid holdup in the catch pot can be calculated and the residue circulating amount and the discharging amount can be regulated based on the result. The fact that the residue circulating amount can be regulated makes it possible to stably operate the depolymerization reactor 15. In addition, it is preferable to provide flow meters in the oligomer feed pipe, the reaction solution feed pipe, the reflux pipe, and the drainage pipe, since this can facilitate the regulation of the flow rates of the reaction solution and the residue and enables stable operation of the depolymerization reactor 15. The catch pot 16 may be further provided with a thermometer.

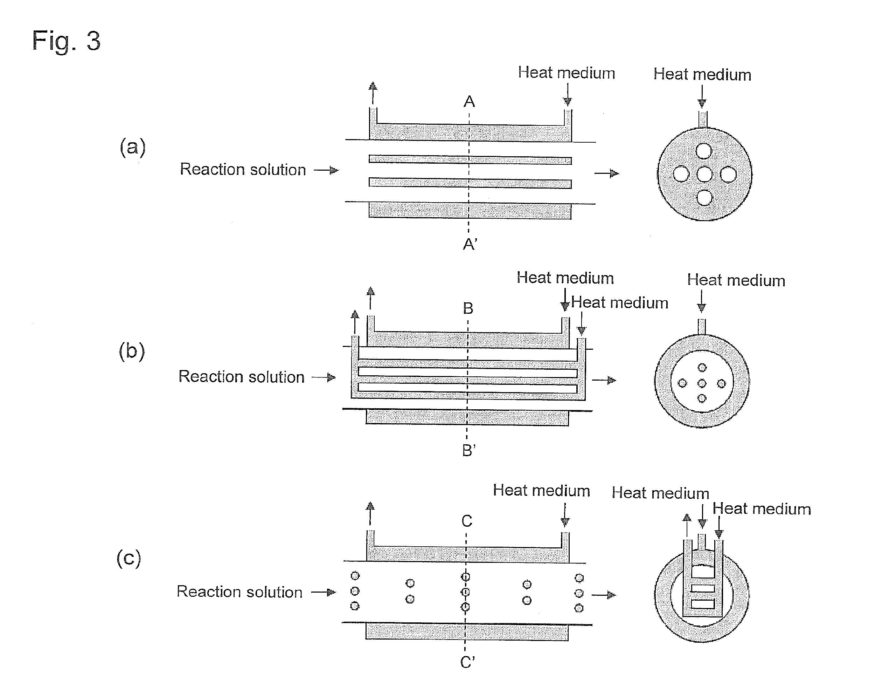

Fig. 3 (a) to (c) show the examples of the disposition of reaction solution passages and heat medium passages in the depolymerization reactor 15. In Fig. 3 (a) to (c), shaded portions correspond to the heat medium passages and the other white portions correspond to the reaction solution passages. On the right side in each figure, the cross section of a site indicated by A-A', B-B', or C-C' in each figure. In any of these embodiments, the reaction solution passages are provided horizontally.

In the embodiment of Fig. 3 (a), a plurality of reaction solution passages having a small inner diameter are provided in parallel within a heat medium passage having a large inner diameter. Specifically, the heat medium passage is provided so that it encloses individual reaction solution passages. In the embodiment of Fig. 3 (b), a plurality of heat medium passages having a small inner diameter are provided within a reaction solution passage having a large inner diameter. The heat medium passages within the reaction solution passage are provided in parallel with the flow direction of a reaction solution. Also, a heat medium passage is also provided in the external part of the reaction solution passage. In the embodiment of Fig. 3 (c), a plurality of heat medium passages having a small inner diameter are provided within a reaction solution passage having a large inner diameter. The heat medium passages within the reaction solution passage are provided vertical (in a direction perpendicular to the flow direction of the reaction solution) to the flow direction of the reaction solution. Also, heat medium passages are provided in the external part of the reaction solution passage.

A heat medium passage(s) may be provided either in parallel or vertical to the flow direction of a reaction solution. When pressure to be applied to a reaction solution is further reduced, a heat medium passage(s) is preferably provided in parallel to the flow direction. On the other hand, when heat transfer efficiency is increased, a heat medium passage(s) is preferably provided vertical to the flow direction of a reaction solution. Alternatively, a heat medium passage(s) may be provided in parallel or at an angle other than vertical to the flow direction of a reaction solution, as necessary. Fig. 3 (a) to (c) merely illustrate the embodiments of reaction solution passages and heat medium passages in the depolymerization apparatus of the present invention. The present invention is not limited to these embodiments.

In the depolymerization reactor 15 having the above configuration, reaction solution passages and heat medium passages are disposed so that they come into contact with each other with a large contact area. This enables efficient heat transfer from a heat medium to a reaction solution and sufficient supply of reaction heat and the heat lost by lactide evaporation. This makes it possible to maintain the temperature of the reaction solution, leading to improved yield and the stable operation of the depolymerization apparatus.

The depolymerization apparatus of the present invention is advantageous in that it can be scaled up relatively easily. For example, when a conventional tank-type depolymerization reactor is used, the liquid depth increases as the apparatus is scaled up, so that pressure to be applied to the reaction solution also increases. When pressure is applied to a reaction solution, there are concerns about matters such as decreased lactide yield and lowered optical purity. However, reaction solution passages are provided horizontally in the depolymerization apparatus of the present invention, so that pressure will never applied excessively to a reaction solution, causing no such problem. Also, the depolymerization apparatus of the present invention has relatively a simple configuration, so that a large-scale apparatus thereof can be easily produced. Furthermore, the depolymerization apparatus of the present invention is appropriate for continuously performing depolymerization.

The present invention also relates to a method for synthesizing polyhydroxycarboxylic acid. The method for synthesizing polyhydroxycarboxylic acid of the present invention comprises a depolymerization step for depolymerizing hydroxycarboxylic acid oligomers to produce hydroxycarboxylic acid cyclic dimers, wherein, in the depolymerization step, a reaction solution is heated by heat transfer from a heat medium passage under reduced pressure while the reaction solution is being flowing through a horizontally provided reaction solution passage. In the depolymerization step of the method, a residual liquid discharged from an outlet of a reaction solution passage is recovered in a catch pot, and at least a portion thereof is refluxed to the reaction solution passage. This is preferable in view of improvement of yield. The method for synthesizing polyhydroxycarboxylic acid of the present invention can be implemented using the above system for synthesizing polyhydroxycarboxylic acid of the present invention.

### Examples

The present invention will be explained more specifically with reference to examples, but the present invention is not limited to the examples.

### [Example 1]

Polylactide was produced using a system for producing polylactide shown in Fig. 1. A lactic acid oligomer having a number average molecular weight of 630 was added as a raw material to a depolymerization reactor 15. The embodiment of a reaction solution passage and heat medium passages shown in Fig. 3 (b) was employed for the depolymerization reactor 15. The space within the reaction solution passage was depressurized to 10 torr or less at 200°C. Tin 2-ethylhexanoate with a concentration of 0.7 kg/m³ was used as a catalyst for a depolymerization reaction. The retention time of a lactic acid oligomer within the depolymerization reactor 15 was determined to be 15 minutes and the liquid depth within the reaction solution passage was set to be 55 cm. In addition, the lactic acid oligomer retention time was defined by dividing the feed flow-rate of the melted oligomer by the amount (retention amount) of melted oligomer in the depolymerization reactor 15, when the feed flow-rate of melted oligomer was equal to the flow rate of condensate obtained from vapor discharged from the depolymerization reactor 15 and the liquid depth within the reaction solution passage was stabilized.

A reaction in a ring-opening polymerization apparatus 24 was performed at 200°C by depressurization to 10 torr or less. The concentration of the polymerization initiator was 700 ppm. The weight average molecular weight of the thus obtained polylactide was about 200,000. The yield based on raw material was 56%.

### [Comparative Example 1]

A lactic acid oligomer having a number average molecular weight of 630, which was the same as that used in Example 1, was used as a raw material. Polylactide was similarly produced using a conventional vertical tank-type depolymerization reactor (liquid depth: 5 m) instead of the depolymerization reactor of the present invention. The weight average molecular weight of the thus obtained polylactide was about 200,000. The yield based on raw material was 43%.

All references, including any publications, patents or patent applications cited in this specification are hereby incorporated by reference in their entirely.

## Claims

1. A method for synthesizing polyhydroxycarboxylic acid comprising a depolymerization step for depolymerizing hydroxycarboxylic acid oligomers to produce hydroxycarboxylic acid cyclic dimers, wherein,
in the depolymerization step, a reaction solution is heated by heat transfer from a heat medium passage under reduced pressure while the reaction solution is being flowing through a horizontally provided reaction solution passage.

2. The method for synthesizing polyhydroxycarboxylic acid according to claim 1, wherein, in the depolymerization step, the remaining solution that is discharged from an outlet of the reaction solution passage is recovered in a catch pot (16) and at least a portion of the solution is refluxed to the reaction solution passage.

3. A polyhydroxycarboxylic acid synthesis system comprising a depolymerization apparatus for depolymerizing hydroxycarboxylic acid oligomers to produce hydroxycarboxylic acid cyclic dimers, wherein
the depolymerization apparatus has a horizontally provided reaction solution passage, a heat medium passage that is in contact with the reaction solution passage, and a depressurization apparatus (33) for depressurizing the reaction solution passage.

4. The polyhydroxycarboxylic acid synthesis system according to claim 3, wherein, in the depolymerization apparatus, a plurality of reaction solution passages are provided in parallel, and the heat medium passage is provided so as to enclose the individual reaction solution passages.

5. The polyhydroxycarboxylic acid synthesis system according to claim 3, wherein, in the depolymerization apparatus, a plurality of heat medium passages are provided within the reaction solution passage.

6. The polyhydroxycarboxylic acid synthesis system according to any one of claims 3 to 5, wherein, in the depolymerization apparatus, the reaction solution passages and the heat medium passages are provided in parallel.

7. The polyhydroxycarboxylic acid synthesis system according to any one of claims 3 to 6, wherein, in the depolymerization apparatus, a catch pot (16) equipped with a liquid-level meter is provided at an outlet of the reaction solution passage.

8. The polyhydroxycarboxylic acid synthesis method according to claim 1 or 2, wherein the system according to any one of claims 3 to 7 is used.

9. A system for producing hydroxycarboxylic acid cyclic dimers, having a horizontally provided reaction solution passage, a heat medium passage that is in contact with the reaction solution passage, and a depressurization apparatus for depressurizing the reaction solution passage.

10. The system for producing hydroxycarboxylic acid cyclic dimers according to claim 9, wherein a plurality of reaction solution passages are provided in parallel, and a heat medium passage is provided so as to enclose the individual reaction solution passages.

11. The system for producing hydroxycarboxylic acid cyclic dimers according to claim 9, wherein a plurality of the heat medium passages are provided within the reaction solution passage.

12. The system for producing hydroxycarboxylic acid cyclic dimer according to any one of claims 9 to 11, wherein the reaction solution passages and the heat medium passages are provided in parallel.

13. The system for producing hydroxycarboxylic acid cyclic dimers according to any one of claims 9 to 12, wherein a catch pot (16) equipped with a liquid-level meter is provided at an outlet of the reaction solution passage.
